# EUROPEAN PATENT APPLICATION

(11) **EP 4 768 912 A1**
(43) Date of publication of application: **01.07.2026**
(21) Application number: 24859674.4
(22) Date of filing: 26.08.2024
(51) Int. Cl.: G01N 33/531, G01N 33/543

(54) **IMMUNOLOGICAL MEASUREMENT METHOD, REAGENT FOR IMMUNOLOGICAL MEASUREMENT, SPECIMEN PRETREATMENT LIQUID FOR IMMUNOLOGICAL MEASUREMENT, REAGENT KIT FOR IMMUNOLOGICAL MEASUREMENT, AND NON-SPECIFIC REACTION INHIBITOR**

(30) Priority: 25.08.2023 US 202363534585 P
(71) Applicant: Sekisui Medical Co., Ltd., Tokyo 103-0027 (JP)
(72) Inventor: FUJIMURA Kengo, Tokyo 103-0027 (JP)
(74) Representative: Ter Meer Steinmeister & Partner
(86) International application number: PCT/JP2024/030136
(87) International publication number: WO 2025/047641

(57) **Abstract**

An immunological measurement method for measuring a measurement target substance in a sample, wherein an antigen-antibody reaction is conducted at least once or more in the presence of an enzyme that specifically degrades Immunoglobulin M, abbreviated as IgM.

## Description

### Technical Field

The present invention relates to an immunological measurement method, a reagent for immunological measurement, a specimen pretreatment liquid for immunological measurement, and a reagent kit for immunological measurement, a non-specific reaction inhibitor. Priority is claimed on Provisional Application 63/534,585 filed in the U.S. on August 25, 2023, the content of which is incorporated herein by reference.

### Background Art

Examples of measurement methods used in the field of diagnostic drugs include immunological measurement methods, which measure a measurement target substance present in a biological sample by utilizing an antigen-antibody reaction. By utilizing antigen-antibody reactions, immunological measurement methods exhibit extremely high specificity.

Biological samples contain all manner of substances, and measurement errors can occur when substances other than the measurement target substance cause non-specific binding reactions or interfere with specific antigen-antibody reactions. These types of phenomena are known as non-specific reactions, and substances that cause such non-specific reactions are referred to as non-specific factors.

Heterophilic antibodies and rheumatoid factors (RF) have been clearly identified as non-specific factors. Heterophilic antibodies is a generic term used for human antibodies that exhibit reactivity with animal-derived antibodies that represent the main reactions used in immunological measurement methods, and human anti-mouse immunoglobulin antibodies (HAMA) are known representative examples. Rheumatoid factors are glycoproteins that are frequently detected in patients with collagen diseases such as rheumatoid arthritis, chronic infections and liver disease, and exhibit reactivity with animal-derived antibodies in a similar manner to HAMA. Rheumatoid factors are known to be human immunoglobulin G or immunoglobulin M (hereinafter abbreviated as IgM) (Non-Patent Documents 1 and 2).

Known techniques for inhibiting non-specific reactions in immunological measurement methods include, for example, those disclosed in Patent Documents 1, 2 and 3.

Patent Document 1 discloses a method for inhibiting non-specific reactions caused by RF by pretreating a sample with a sufficient amount of an animal-derived antibody having a binding ability with the antigen-binding site (Fab) of human rheumatoid factor. Examples of these types of animal-derived antibodies include anti-human immunoglobulin Fab antibodies, anti-human IgG antibodies (Fab specific), anti-human IgA antibodies (Fab specific), and anti-human IgM antibodies (Fab specific).

Patent Document 2 discloses a method for inhibiting non-specific reactions by adding a polyclonal antibody against IgM natural antibodies prepared from the same type of animal as the antibody used in the measurement.

Patent Document 3 discloses a method for inhibiting non-specific reactions caused by interfering substances such as rheumatoid factors that have a structure in which polypeptide chains are bound together by disulfide bonds, by using a reducing agent to cleave the disulfide bonds, thereby degrading the interfering substance.

### Citation List

### Patent Documents

Patent Document 1: Japanese Unexamined Patent Application, First Publication No. Hei 07-012818
Patent Document 2: Japanese Unexamined Patent Application, First Publication No. Hei 11-287801
Patent Document 3: Japanese Unexamined Patent Application, First Publication No. Hei 13-255325

### Non-Patent Documents

Non-Patent Document 1: Japanese Journal of Clinical Chemistry, Volume 23 supplement, 175a-1 to 175a-10 (1994)
Non-Patent Document 2: Immunological Testing, Tetsuo Kubota, Kiyotaka Fujita, Eiji Hosoi, and Michiko Kajiwara, Ishiyaku Publishing, Inc., page 199

### Summary of Invention

### Technical Problem

The anti-human IgM polyclonal antibodies, anti-human IgG polyclonal antibodies and anti-human IgA polyclonal antibodies described in Patent Document 1 are currently used in a variety of reagents to suppress non-specific reactions caused by natural antibodies and RF, but it became evident that this method is unable to satisfactorily suppress non-specific reactions.

The polyclonal antibody against IgM natural antibodies described in Patent Document 2 must be prepared from the same type of animal as the antibody used for measurement, and there were limitations on the preparation method and the antibody against the measurement target substance.

The reducing agent described in Patent Document 3, which is used for inhibiting non-specific reactions caused by interfering substances having a structure in which polypeptide chains are bound together with disulfide bonds, may sometimes, depending on the type or concentration, cleave disulfide bonds in the antigen or antibody that represents the measurement target substance or in antigens or antibodies included as reagent components, and therefore lacks versatility. Furthermore, methods using reducing agents may also degrade IgG and IgA as well as IgM. Accordingly, this method could not be used, particularly when human IgG is the measurement target substance.

An object of the present invention is to provide an immunological measurement method that is capable of inhibiting non-specific reactions caused by IgM contained in the measurement sample.

### Solution to Problem

In order to achieve the above object, the inventors of the present invention investigated the non-specific reaction inhibitory effect of a variety of substances, and as a result, discovered that by conducting an antigen-antibody reaction in the presence of an enzyme that specifically degrades IgM, non-specific reactions were able to be suppressed, enabling them to complete the present invention. Specifically, the present invention has the following aspects.

[1] An immunological measurement method for measuring a measurement target substance in a sample, wherein an antigen-antibody reaction is conducted at least once or more in the presence of an enzyme that specifically degrades Immunoglobulin M.
[2] The immunological measurement method according to [1], wherein the enzyme is a protease.
[3] The immunological measurement method according to [1] or [2], wherein the protease is a proteinase or a peptidase.
[4] The immunological measurement method according to any of [1] to [3], wherein the enzyme is an enzyme that cleaves a portion of the IgM constant region including CHµ1 to CHµ4.
[5] The immunological measurement method according to any of [1] to [4], wherein the enzyme is an enzyme that acts on the IgM in the sample and degrades the IgM into a F(ab')₂ fragment and a Fc fragment.
[6] The immunological measurement method according to any of [1] to [5], wherein the immunological measurement method is a latex turbidimetric immunoassay method.
[7] The immunological measurement method according to any of [1] to [6], wherein in the immunological measurement method, the measurement target substance is an antigen or antibody.
[8] A reagent for immunological measurement used in an immunological measurement method for measuring a measurement target substance in a sample, wherein the reagent contains an enzyme that specifically degrades IgM.
[9] The reagent for immunological measurement according to [8], wherein the enzyme is a protease.
[10] The reagent for immunological measurement according to [8] or [9], wherein the protease is a proteinase or a peptidase.
[11] The reagent for immunological measurement according to any of [8] to [10], wherein the enzyme is an enzyme that cleaves a portion of the IgM constant region including CHµ1 to CHµ4.
[12] The reagent for immunological measurement according to any of [8] to [11], wherein the enzyme is an enzyme that acts on the IgM in the sample and degrades the IgM into a F(ab')₂ fragment and a Fc fragment.
[13] The reagent for immunological measurement according to any of [8] to [12], wherein the reagent for immunological measurement is a reagent for use in a latex turbidimetric immunoassay method.
[14] The reagent for immunological measurement according to any one of [8] to [13], wherein the measurement target substance in the immunological measurement method is an antigen or antibody.
[15] A specimen pretreatment liquid for immunological measurement containing an enzyme that specifically degrades IgM.
[16] The specimen pretreatment liquid for immunological measurement according to [15], wherein the enzyme is a protease.
[17] The specimen pretreatment liquid for immunological measurement according to [15] or [16], wherein the protease is a proteinase or a peptidase.
[18] The specimen pretreatment liquid for immunological measurement according to any of [15] to [17], wherein the enzyme is an enzyme that cleaves a portion of the IgM constant region including CHµ1 to CHµ4.
[19] The specimen pretreatment liquid for immunological measurement according to any of [15] to [18], wherein the enzyme is an enzyme that acts on the IgM in the sample and degrades the IgM into a F(ab')₂ fragment and a Fc fragment.
[20] The specimen pretreatment liquid for immunological measurement according to any of [15] to [19], wherein the specimen pretreatment liquid for immunological measurement is a liquid for use in a latex turbidimetric immunoassay method.
[21] A reagent kit for immunological measurement used in an immunological measurement method for measuring a measurement target substance in a sample, wherein the reagent kit contains an enzyme that specifically degrades IgM.
[22] The reagent kit for immunological measurement according to [21], wherein the enzyme is a protease.
[23] The reagent kit for immunological measurement according to [21] or [22], wherein the protease is a proteinase or a peptidase.
[24] The reagent kit for immunological measurement according to [22] or [23], wherein the enzyme is an enzyme that cleaves a portion of the IgM constant region including CHµ1 to CHµ4.
[25] The reagent kit for immunological measurement according to any of [22] to [24], wherein the enzyme is an enzyme that acts on the IgM in the sample and degrades the IgM into a F(ab')₂ fragment and a Fc fragment.
[26] A non-specific reaction inhibitor containing an enzyme that specifically degrades IgM.

### Advantageous Effects of Invention

The present invention is able to provide an immunological measurement method that is capable of inhibiting non-specific reactions caused by IgM contained in the measurement sample.

The immunological measurement method of the present invention is able to inhibit non-specific reactions that have not been able to be inhibited using conventional non-specific reaction inhibitors. As a result, accurate measurements of the measurement target substance became possible.

### Description of Embodiments

### [Immunological Measurement Method]

The immunological measurement method of the present invention is a method for immunologically measuring a measurement target substance in a sample, wherein an antigen-antibody reaction is conducted at least once or more in the presence of an enzyme that specifically degrades IgM. In other words, this is a method in which an enzyme that specifically degrades IgM is reacted with the sample as a non-specific reaction inhibitor, and in the presence of that enzyme, the measurement target substance in the sample is measured immunologically using a specific binding partner.

One aspect of the immunological measurement method of the present invention is a non-specific reaction inhibition method, wherein during the immunological measurement method, an antigen-antibody reaction is conducted at least once or more in the presence of the enzyme that specifically degrades IgM, and at this time, non-specific reactions in the reaction liquid are inhibited.

Immunological measurement methods can be broadly classified into homogeneous methods and heterogeneous methods.

Homogeneous methods are measurement methods in which a binding reaction that occurs between the measurement target substance and a specific binding partner in a mixed solution (reaction liquid) of the sample and a reagent liquid is detected specifically without requiring a B/F (bound/free) separation. Heterogeneous methods are measurement methods in which a B/F separation operation is conducted, excess components that have not participated in binding reactions are washed away and removed, and a binding reaction is then progressed to detect the measurement target substance.

Heterogeneous methods require a washing step, meaning the number of steps is greater and more time is required for conducting measurements, but offer the advantage of being comparatively unaffected by non-specific reaction substances. In contrast, homogeneous methods do not employ a washing step, and therefore tend to be more easily affected by non-specific reactions, but are simple with few steps, and require little time for conducting measurements, and are therefore widely requested in the field of clinical diagnosis.

Examples of homogenous methods include measurement methods that utilize an immunoagglutination assay (IA) method, and specific examples include turbidimetric immunoassay (TIA) methods and immunochromatography methods (lateral flow and flow-through). TIA is a method for qualitatively or quantitatively detecting an analyte (measurement target substance) in a sample based on the degree of agglutination of immunoconjugates formed by crosslinking of the analyte caused by a specific binding partner such as an antibody. Among TIA methods, the latex turbidimetric immunoassay (LTIA) method (hereinafter sometimes abbreviated as LTIA), which uses latex particles as an insoluble carrier to amplify the agglutination signal, is suited to optical detection and can be easily automated, and is therefore a highly versatile measurement method that can be applied to a variety of test items.

Examples of heterogeneous methods include the ELISA method using a well plate, and chemiluminescence methods.

The present invention can be used with any of the above immunological measurement methods, but homogeneous methods, which are comparatively more susceptible to the effects of non-specific reactions, can be expected to exhibit a greater effect and are consequently preferred, and among the various homogeneous methods, the latex turbidimetric immunoassay method is the most desirable.

### [Enzyme that Specifically Degrades IgM]

One aspect of the present invention is a method for conducting immunological measurements using an enzyme that specifically degrades IgM, or a non-specific reaction inhibitor containing that enzyme (hereinafter, the enzyme that specifically degrades IgM is sometimes referred to as the IgM-specific degrading enzyme). The IgM-specific degrading enzyme may be any enzyme that specifically degrades IgM, and examples include proteases. Among proteases, more specific examples include proteinases (endopeptidases), and peptidases (exopeptidases) and the like. In the present invention, the enzyme that degrades IgM is preferably an enzyme that cleaves a portion of the IgM constant region including C_{H}µ1 to C_{H}µ4 (for example, the constant region of human IgM from Ser124 to Tyr576: SEQ ID NO: 1), and is more preferably capable of degrading IgM into F(ab')₂ and Fc fragments. The IgM-specific degrading enzyme need only have the function of specifically degrading IgM when brought into contact with a sample such as a specimen, and the enzyme may be inactivated by the time the subsequent antigen-antibody reaction is conducted.

In this description, the expression that an antibody "reacts" with an antigen, and the expression that an antibody "recognizes" an antigen are used with the same meaning, but these expressions are not limited to these examples, and should be interpreted in the broadest sense. Confirmation as to whether or not an antibody "reacts" with an antigen can be made by the antigen-immobilized ELISA method, the competitive ELISA method, or the sandwich ELISA method or the like, or by a method that utilizes the principle of surface plasmon resonance (namely, the SPR method). The SPR method can be conducted using an apparatus, sensor, and reagents commercially available under the name Biacore (a registered trademark).

In this description, the expressions that an enzyme "degrades" IgM, that an enzyme "reacts" with IgM, and that an enzyme "digests" IgM are used with the same meaning, but these expressions are not limited to these examples, and the meaning of the expression that an enzyme "degrades" IgM, should be interpreted in the broadest sense.

The IgM-specific degrading enzyme in the present invention specifically degrades IgM. The IgM-specific degrading enzyme degrades IgM more efficiently than immunoglobulins other than IgM such as IgG and IgA. As a result, even in those cases where the measurement target substance is IgG, or in cases where the binding partner that specifically binds to the measurement target substance is IgG, the IgM-specific degrading enzyme can be used without affecting the specific measurement of the measurement target substance. Furthermore, IgM-specific degrading enzymes can degrade IgM derived from organisms such as humans, mice, rats, rabbits, goats, ostriches and pigs, and in those cases where a specific degradation of human IgM is required, a human IgM-specific degrading enzyme is preferably used.

In the present invention, the expression that the IgM-specific degrading enzyme specifically degrades IgM means that, for example, after reacting IgG or IgM with the enzyme at 37°C for a period of 30 to 60 minutes, the amount of residual IgM compared with the amount of IgG is not more than 50%, not more than 40%, or 30% or less, is preferably 20% or less, and is more preferably 10% or less.

The IgM-specific degrading enzyme used in the present invention may be any form of enzyme, including naturally occurring enzymes and recombinant enzymes produced using gene recombination techniques. Examples of naturally occurring enzymes include enzymes produced by various bacteria and fungi, such as those produced by streptococci, Legionella bacteria, and Candida. Preferred examples include the immunoglobulin M-degrading enzyme of S. suis (Ig d_{Ssuis}) produced by Streptococcus suis (Seele et al., J. Bacteriol., 2013; 195, 930 to 940) or amino acid variants of that enzyme, as well as IgMBRAZOR (manufactured by Genovis Inc.) or amino acid variants of that enzyme. Further, the above enzyme may also be a recombinant enzyme produced as a recombinant protein. Modified recombinant enzymes having the desired enzyme activity can also be produced by artificially introducing amino acid residue substitutions into a recombinant enzyme, and then conducting screening using various known assay systems (Japanese Unexamined Patent Application, First Publication No. 2019-506866, EP 3148576). Accordingly, in the present invention, various IgM-specific degrading enzymes can be used to implement non-specific reaction inhibition in the immunological measurement method, or to produce a non-specific reaction inhibitor.

In the present invention, examples of the method for incorporating the enzyme that specifically degrades IgM into an antigen-antibody reaction system include methods in which the enzyme is used as one reagent constituent of a reagent for immunological measurement, and methods in which the enzyme is added to a specimen dilute solution, a specimen extract, or a specimen pretreatment liquid or the like. For example, a specimen dilute solution, specimen extract, or specimen pretreatment liquid already containing the IgM-specific degrading enzyme may be used, or the IgM-specific degrading enzyme may be added to the specimen dilute solution, specimen extract, or specimen pretreatment liquid. In this description, the term specimen pretreatment liquid is deemed to include both specimen dilute solutions and specimen extracts.

The term "antigen-antibody reaction system", for example, in the case where the reagent for immunological measurement is a liquid reagent, refers to a liquid phase in which the sample and the reagent for immunological measurement are mixed, and the antigen-antibody reaction occurs.

For example, in the case of the LTIA method, the sample may be mixed with a reagent for immunological measurement that includes an IgM-specific degrading enzyme, or the sample may be mixed in advance with a specimen pretreatment liquid containing an IgM-specific degrading enzyme, and the resulting mixture then mixed with the LTIA measurement reagent.

In the case of the ELISA method, the sample may be mixed in advance with the IgM-specific degrading enzyme, and the resulting mixture then added dropwise onto the microplate, or the sample may be mixed with a solution containing the IgM-specific degrading enzyme and the detection antibody, and the resulting mixture then added dropwise onto the microplate.

In the case of a reagent for a chemiluminescence method, the sample may be mixed in advance with a specimen pretreatment liquid containing the IgM-specific degrading enzyme, and the resulting mixture then mixed with the reagent for immunological measurement, or the IgM-specific degrading enzyme may be incorporated in the reagent for immunological measurement (for example, a solution containing the detection antibody or detection antigen and magnetic particles).

In those cases where the antigen-antibody reaction is carried out in the solid phase, such as an immunochromatography method, the "antigen-antibody reaction system" refers to the solid phase in which the antigen-antibody reaction occurs between a liquid sample and a binding partner. In such cases, the sample may be mixed in advance with a specimen pretreatment liquid containing the IgM-specific enzyme, and the resulting mixture then added dropwise onto the immunochromatographic test piece, or the IgM-specific enzyme may be dried and held on a member such as a sample pad (sample supply site), so that when the sample is added dropwise thereto, the IgM-specific enzyme is dissolved and develops the solid phase, thereby reaching a state of being present in the reaction system.

In the present invention, the concentration of the IgM-specific degrading enzyme may be any concentration that does not significantly affect the antigen-antibody reaction between the measurement target substance and the specific binding partner, but is capable of exerting the desired non-specific reaction inhibitory effect, and may be set appropriately by a person skilled in the art in accordance with the type of measurement target substance and the type of sample.

The concentration of the IgM-specific degrading enzyme in the antigen-antibody reaction system also varies depending on the reagent composition in the antigen-antibody reaction system. For example, in those cases where the IgM-specific enzyme is added to a specimen pretreatment liquid, the enzyme may be added in an amount within a range from 1 to 1000 U, preferably from 5 to 800 U, more preferably from 10 to 500 U, even more preferably from 20 to 300 U, and most preferably from 50 to 200 U, per 10 µL of the specimen. Further, in those cases where the IgM-specific enzyme is added to a specimen pretreatment liquid, the enzyme concentration in the pretreatment liquid may be within a range from 1 to 1000 U/µL, preferably from 5 to 800 U/µL, more preferably from 10 to 500 U/µL, even more preferably from 10 to 300 U/µL, and most preferably from 20 to 200 U/µL. Furthermore, when the IgM-specific enzyme is added to a specimen pretreatment liquid, the mixing ratio between the specimen and the specimen pretreatment liquid may be within a range from 1:100 to 100:1, preferably from 1:50 to 50:1, even more preferably from 1:20 to 20:1, and most preferably from 1:10 to 10:1.

In the present invention, the IgM-specific degrading enzyme may be used alone, or may be used in combination with other substances having a non-specific reaction inhibitory effect. Examples of other substances having a non-specific reaction inhibitory effect include anti-IgM antibodies, polymer compounds, and modified antibodies in which a portion or all of the variable region of the L chain or H chain of a specific antibody has been modified. These other substances having a non-specific reaction inhibitory effect are not limited to those listed here. Furthermore, when an anti-IgM antibody and an IgM-specific degrading enzyme are used in combination, the anti-IgM antibody and the enzyme may exist in a state where they are bound together, either directly or indirectly.

In those cases where the IgM-specific degrading enzyme is incorporated in advance in the reagent for immunological measurement according to the present invention, the enzyme is preferably incorporated in advance in the measurement reagent in an amount sufficient to achieve a concentration in the reaction system that satisfies the value described above.

### [Latex Turbidimetric Immunoassay Method (LTIA Method)]

The LTIA method, which represents one example of the immunological measurement method of the present invention, is described below. Method for measuring a measurement target substance using the LTIA method can be broadly classified into two types.

The first type describes methods in which latex particles onto which a specific binding partner for the measurement target substance has been immobilized are reacted with the measurement target substance to form a sandwich-type immunoconjugate, and the measurement target substance is measured based on the degree of agglutination of the latex particles that accompanies the formation of the immunoconjugate.

The second type describes methods in which a protein or the like onto which a plurality of measurement target substances or analogs thereof (including fragments thereof) have been immobilized is added to the reagent for immunological measurement, causing competition between these and the measurement target substance in the sample and inhibiting the formation of an immunoconjugate between the measurement target substance contained in the reagent and latex particles onto which a specific binding partner for the measurement target substance has been immobilized, and the measurement target substance (for example, an antigen) is then measured based on the degree of inhibition of agglutination of the latex particles accompanying the inhibition of immunoconjugate formation.

The measurement target substance and the specific binding partner for the measurement target substance may be any of proteins, peptides, sugar chains, lipids, glycoproteins, glycolipids, nucleic acids, low-molecular weight compounds, and high-molecular weight compounds, provided specific binding with the measurement target substance can occur, and any substance may be selected depending on the intended purpose. For example, if the measurement target substance is an antigen, any antibody such as a polyclonal antibody or monoclonal antibody (including monoclonal antibodies produced from hybridomas, gene recombinant antibodies, and functional fragments of various antibodies) may be selected as the specific binding partner for the measurement target substance. If the measurement target substance is an antibody, an antigen such as a natural antigen or gene recombinant antigen may be selected as the specific binding partner for the measurement target substance.

The present invention may be used in any of the methods described above, but specifically, may be used in the following steps, although the present invention is not limited to these steps.

(1) A step of bringing a sample containing a measurement target substance and an IgM-specific degrading enzyme into contact in solution.
(2) Following step (1), a step of adding latex particles onto which a specific binding partner for the measurement target substance has been supported to the solution.
(3) Following step (2), a step of optically detecting the degree of agglutination of the latex particles in the solution.

Here, step (3) means "a step of measuring the agglutination reaction between the measurement target substance and the latex particles without conducting a washing and separation step, either during step (2) or following step (2)."

In the LTIA method, the measurement target substance can be measured by optically observing the degree of agglutination that has occurred. Examples of optical observation methods include methods (such as endpoint methods and rate methods) for measuring the scattered light intensity, absorbance, or transmitted light intensity using optical equipment. The measured value for the absorbance or the like obtained by measuring the sample is compared with the measured values for the absorbance or the like obtained upon measurement of standard substances (samples with known concentrations of the measurement target substance), thereby calculating the concentration (quantitative value) of the measurement target substance contained in the sample. The measurement of the absorbance or the like of transmitted light or scattered light or the like may be a one-wavelength measurement or a two-wavelength measurement (using the difference or ratio between the two wavelength values). The measurement wavelength is generally selected from within a range from 500 nm to 900 nm.

In the present invention, the measurement of the measurement target substance in the sample may be conducted manually, or may be conducted using a device such as a measurement device. The measurement device may be a general-purpose automatic analyzer or a special-purpose measurement device (dedicated device). Furthermore, this measurement is preferably conducted using a method involving multiple operational steps, such as a two-step method (two-reagent method).

### [Latex Particles having Specific Binding Partner Supported Thereon]

The specific binding partner for the measurement target substance may be immobilized and supported on the latex particles by a conventional method such as a physical adsorption method, a chemical binding method, or a combination thereof. In the case of a physical adsorption method, the method may be conducted in accordance with a conventional method, by mixing and contacting the specific binding partner for the measurement target substance and the latex particles in a solution such as a buffer solution, or by bringing a solution of the specific binding partner for the measurement target substance dissolved in a buffer solution or the like into contact with a carrier. Further, in the case of a chemical binding method, the specific binding partner for the measurement target substance and the carrier may be mixed and brought into contact with a divalent crosslinking reagent such as glutaraldehyde, a carbodiimide, an imide ester or a maleimide, in accordance with a known method disclosed in "Clinical Pathology Extra Supplementary Special Feature No. 53: Immunoassay for Clinical Testing - Technology and Applications" edited by the Japanese Society of Clinical Pathology, published by Clinical Pathology Publishing Association, 1983; or "New Biochemical Testing Course 1: Protein IV" edited by the Japanese Biochemical Society, published by Tokyo Kagaku Dojin, 1991, thereby reacting the amino groups, carboxyl groups, thiol groups, aldehyde groups, or hydroxyl groups or the like of the specific binding partner for the measurement target substance and the carrier with the divalent crosslinking reagent.

There are no particular limitations on the synthetic polymer that constitutes the latex particles, and examples include polystyrene, styrene-styrene sulfonate copolymers, methacrylic acid polymers, acrylic acid polymers, itaconic acid polymers, styrene-hydrophilic carboxy monomer copolymers such as styrene-methacrylic acid copolymers, styrene-acrylic acid copolymers and styrene-itaconic acid copolymers, and vinylnaphthalene polymers. Among these, styrene-methacrylic acid copolymers, styrene-itaconic acid copolymers, styrene and styrene-styrene sulfonate copolymers are preferred. Styrene and styrene-(meth)acrylic acid copolymers are preferred.

There are preferably a plurality of types of the specific binding partner for the measurement target substance that is supported on the latex particles in order to facilitate sandwich formation. In those cases where a plurality of antibody recognition sites exist on the measurement target substance, a single type of specific binding partner may be used. For example, when the specific binding partner is a monoclonal antibody, a plurality of monoclonal antibodies with different recognition sites are used. Further, for example, when the specific binding partner is a polyclonal antibody, a polyclonal antibody derived from a single type of antiserum may be used, or the polyclonal antibody may be derived from a plurality of types of antiserum. Furthermore, combinations of a monoclonal antibody and a polyclonal antibody may also be used.

If a treatment is required to suppress spontaneous agglutination of the latex particles or non-specific reactions or the like, then a carrier blocking treatment (masking treatment) may be conducted by treating the surface of the latex particles using a known method such as surface coating by contact with a protein such as bovine serum albumin (BSA), casein, gelatin, egg white albumin or a salt thereof, a surfactant, or skim milk powder or the like.

### [Reagent for Immunological Measurement]

The immunological measurement method of the present invention can be conducted using a reagent for immunological measurement. This reagent for immunological measurement contains the main components for the antigen-antibody reaction, and the IgM-specific degrading enzyme described above. Examples of the above main components include specific binding partners for the measurement target substance, as well as insoluble carriers such as particles for immunological measurement, immunochromatographic test pieces, and microplates.

The reagent for immunological measurement according to the present invention may also contain buffers, proteins, peptides, amino acids, nucleic acids, lipids, phospholipids, sugars, glycoproteins, glycolipids, inorganic salts, polymer compounds, surfactants, other non-specific reaction inhibitors, and preservatives and the like in amounts that do not interfere with the non-specific reaction inhibitory effect of the IgM-specific degrading enzyme. Examples of components that may be included in order to buffer and adjust the pH, the ionic strength, and/or the osmotic pressure or the like of the sample include buffer solutions such as acetic acid, citric acid, phosphoric acid, Tris, glycine, boric acid, carbonic acid, phthalic acid, succinic acid, maleic acid and imidazole, Good's buffer, and sodium salts, potassium salts, and calcium salts and the like of these buffers. Furthermore, polymers such as polyvinylpyrrolidone and phospholipid polymers may be included as components for enhancing the agglutination formation of particles for immunological measurement.

The IgM-specific degrading enzyme may be included in the constituent reagent in a concentration that enables adjustment to the required concentration within the antigen-antibody reaction system in the mixed state between the reagent and the sample at the time of measurement, and this concentration may vary depending on the type of reagent.

### [Reagent Kit for Immunological Measurement]

The reagent kit for immunological measurement according to the present invention contains at least the IgM-specific degrading enzyme as a kit component. Accordingly, the reagent kit of the present invention contains the IgM-specific degrading enzyme in one, or two or more of the reagent associated with the antigen-antibody reaction that constitutes the kit, the specimen dilution liquid, and the specimen pretreatment liquid containing the specimen extract. In addition to the above, the kit may also include an instruction manual and sample collection tools (collection pipette, syringe, cotton swab, and/or filtration filter or the like).

The reagent compositions employed with various immunological measurement methods are described below.

### [Latex Turbidimetric Immunoassay Method]

Examples of the reagent (LTIA reagent) in those cases where the immunological measurement method is the latex turbidimetric immunoassay method are presented below, but the invention is not limited to the following reagents.

(1) A first reagent containing the IgM-specific degrading enzyme
(2) A second reagent containing latex particles with a specific binding partner for the measurement target substance supported thereon

The first reagent typically contains a buffer solution, and the concentration of the IgM-specific degrading enzyme in the buffer solution may be a concentration that enables adjustment to the aforementioned preferred enzyme concentration in the mixed state between the reagent and the sample at the time of measurement, and varies depending on the type of reagent. The enzyme may also be contained in the second reagent as well as the first reagent.

In the LTIA reagent of one example of the present invention, the concentration of the IgM-specific degrading enzyme contained in the first reagent is typically within a range from 1 to 1,000 µg/mL, preferably from 5 to 500 µg/mL, more preferably from 10 to 100 µg/mL, and even more preferably from 20 to 50 µg/mL, although the concentration is not limited to these values.

In those cases where a specimen pretreatment liquid or the like is added to the specimen prior to supply of the specimen to the LTIA method, the IgM-specific degrading enzyme may be included in the specimen pretreatment liquid, and the concentration of the enzyme within the pretreatment liquid is typically within a range from 1 to 1,000 µg/mL, preferably from 5 to 500 µg/mL, more preferably from 10 to 300 µg/mL, and even more preferably from 20 to 200 µg/mL, although the concentration is not limited to these values.

### (Temperature)

The temperature for the reaction of the IgM-specific degrading enzyme with the specimen may be any temperature at which the IgM-specific degrading enzyme is not inactivated, and IgM is able to be specifically degraded, and the temperature is typically within a range from 0 to 50°C, preferably from 10 to 40°C, and more preferably from 20 to 40°C, although the temperature is not limited to these values.

### (Reaction Time)

The reaction time for the reaction of the IgM-specific degrading enzyme with the specimen may be any time that enables the IgM to undergo sufficient specific degradation using the IgM-specific degrading enzyme, and is typically within a range from 30 seconds to 24 hours, and preferably from 1 to 120 minutes, although the reaction time is not limited to these values.

### (pH)

The pH of the solution containing the IgM-specific degrading enzyme may be any pH that enables the IgM-specific degrading enzyme to function satisfactorily, and is typically within a range from pH 1 to 12, preferably from 3 to 10, more preferably from 5 to 9, and most preferably from 6 to 8, although the pH is not limited to these values.

### (Particles for Immunological Measurement)

Besides the latex particles described above, the particles for immunological measurement used in the present invention may also use any other conventional particles capable of supporting a specific binding partner for the measurement target substance. For example, inorganic particles such as metal colloids, silica, carbon or magnetic particles may also be used as the particles for immunological measurement according to the present invention.

The size of the particles for immunological measurement may be selected with due consideration of the optical measurement method being used (for example, a turbidimetric method that measures transmitted light, or a nephelometry that measures scattered light), and is typically selected appropriately from within a range from 0.05 to 1 µm, so as to achieve the desired measurement sensitivity and measurement range. The average particle size in optical measurements using an automatic analyzer is generally within a range from 0.1 to 0.4 µm, but the particle size is not restricted to these values.

### [ELISA Method]

The ELISA method is a method that utilizes a combination of a variety of antigen-antibody reactions, finally resulting in an enzyme-labeled antigen or antibody being incorporated into the reaction system to detect enzyme activity. The detection of enzyme activity uses a substrate having an absorption spectrum that changes upon reaction, and depending on the combination of antigen-antibody reactions, may employ a direct method, indirect method, sandwich method, or competitive method or the like.

Examples of the reagents for immunological measurement in those cases where the immunological measurement method of the present invention is a sandwich ELISA method are shown below.

(a) Insoluble carriers on which an antibody that reacts with the measurement target substance has been immobilized
(b) Antibodies which are labeled with a labeling substance, and react with the measurement target substance

The insoluble carrier of (a) above is preferably a plate, and the labeling substance may be selected as appropriate. The antibody immobilized on the insoluble carrier captures the measurement target substance in the solution containing the sample, forming a complex on the insoluble carrier. The antibody labeled with a labeling substance binds to the captured measurement target substance and forms a sandwich with the complex. The measurement target substance in the sample can be measured by measuring the amount of the labeling substance using a method appropriate for the labeling substance. Specific details of the methods, such as the method for immobilizing the antibody on the insoluble carrier or the method for binding the antibody to the labeling substance are well known to those skilled in the art, and these methods can be used without any particular limitations.

In the ELISA method, the IgM-specific degrading enzyme of the present invention may be incorporated in the immunological reaction system, for example, by addition to the specimen pretreatment liquid or the like, or by addition to the solution in which the antigen-antibody reaction is conducted.

### [Immunochromatography Method]

The composition of the reagents for immunological measurement (namely, the composition of the test piece) in those cases where the immunological measurement method of the present invention is an immunochromatography method is described below.

### Immunochromatographic Test Piece:

In those cases where an antibody is used as the specific binding partner, the test piece is provided on a sheet-like insoluble carrier such as a porous membrane with "1. a sample supply site", "2. a site for holding the labeled antibody (labeled antibody holding site)" and "3. a site for immobilizing an antibody to capture the complex formed by the labeled antibody and the measurement target substance (capture antibody site)", which are provided in sequence in the direction of development of the solution containing the sample.

In the immunochromatography method, by including at least the type of test piece described above, and adding a prescribed amount of a sample containing the measurement target substance to the sample supply site, the sample is drawn into the labeled antibody holding site by capillary action, and the measurement target substance and the labeled antibody bind together to form a complex. When the complex develops the membrane and enters the antibody capture site, the complex is captured by the antibody (the capture antibody) immobilized on the membrane, and a complex of the capture antibody - the measurement target substance - the labeled antibody is formed. The measurement target substance can then be detected by detecting the label by any appropriate method (for example, in the case of a visible label such as a gold colloid, by using an aggregation image, or in a case of an enzyme, by using a color development reaction by adding a substrate).

In the immunochromatography method, the IgM-specific degrading enzyme of the present invention can be incorporated in the reaction system, for example, by addition to the specimen pretreatment liquid or the like, or by incorporation, drying and holding at the sample supply site or the labeled antibody holding site.

### [Chemiluminescence Method]

The measurement target substance is reacted with magnetic particles having an antigen or an antibody bound thereto to form a complex, and unreacted substances are then removed using magnetism. A reagent containing a labeled antibody is then added, and after unreacted substances are removed by magnetism, a luminescent reagent is added and the amount of luminescence is measured. In those cases where an enzyme is used as the label, the method is referred to as a chemiluminescent enzyme immunoassay (CLEIA method). In those cases where a metal complex such as a ruthenium pyridine complex is used as the label and the luminescence intensity is measured by an electrochemical reaction, the method is referred to as an electro chemiluminescence immunoassay (ECLIA method). In those cases where a chemiluminescent substance is used as the label, the method is referred to as a chemiluminescent immunoassay (CLIA method).

Examples of the reagents for immunological measurement in those cases where the immunological measurement method of the present invention is the CLEIA method are shown below.
(a) Magnetic particles on which are immobilized an antibody (or antigen) that reacts with the measurement target substance
(b) An enzyme-labeled antibody (or antigen) that reacts with the measurement target substance
(c) A luminescent reagent

The antibody immobilized on the magnetic particles captures the measurement target substance in the solution containing the sample, and forms a complex. The antibody labeled with an enzyme-labeled substance binds to the captured measurement target substance to form a sandwich with the above complex. By reacting the enzyme-labeled substance with the luminescent reagent and measuring the amount of luminescence, the measurement target substance within the sample can be measured.

In the CLEIA method, the IgM-specific degrading enzyme of the present invention may be incorporated into the immunological reaction system, for example, by addition to the specimen pretreatment liquid or the like, or by addition to the solution in which the antigen-antibody reaction is conducted.

### [Specific Binding Partner]

In the present invention, the specific binding partner for the measurement target substance may be any substance capable of specifically binding to the measurement target substance, including proteins, peptides, amino acids, lipids, sugars, glycoproteins, glycolipids, nucleic acids, haptens, low-molecular weight compounds, and high-molecular weight compounds. Moreover, there are no particular limitations on the size of the molecular weight or the origin of the specific binding partner, which may be natural or synthetic, and examples include all manner of antibodies or antigens that can be used in immunological measurement methods that utilize antigen-antibody reactions.

The antibodies mentioned above may be polyclonal antibodies or monoclonal antibodies. Monoclonal antibodies are more preferred.

### <Sample>

In the present invention, examples of the sample containing the measurement target substance include human or animal blood, serum, plasma, culture supernatants, urine, cerebrospinal fluid, saliva, sweat, ascites, nasal discharge, feces, or cell or tissue extracts. Blood is the most preferred sample containing the measurement target substance. It is known that the standard values for IgM contained in blood are generally within a range from 33 to 190 mg/dl for men and from 46 to 260 mg/dl for women. The sample is sometimes referred to as a "specimen."

### [Measurement Target Substance]

The reagent for immunological measurement according to the present invention can measure a variety of substances contained within the sample as the measurement target substance. Examples of the measurement target substance include proteins, peptides, amino acids, lipids, sugars, glycoproteins, glycolipids, nucleic acids, and haptens, but there are no particular limitations on the measurement target substance, provided it is a substance that can be theoretically measured. Specific examples include C-reactive protein (CRP), lipoprotein(a) (Lp(a)), matrix metalloproteinase 3 (MMP3), antiphospholipid antibodies, type IV collagen, prostate specific antigen (PSA), brain natriuretic peptide (BNP), insulin, albumin, cystatin C, rheumatoid factor (RF), KL-6, procalcitonin (PCT), fibrin and fibrinogen degradation products (FDP), D-dimer, soluble fibrin (SF), thrombin-antithrombin III complex (TAT), transferrin, haptoglobin, α1-antitrypsin, α1-acidoglycoprotein, α2-macroglobulin, hemopexin, antithrombin III, α-fetoprotein, carcinoembryonic antigen (CEA), ferritin, hepatitis B virus envelope s antigen (HBs-Ag), anti-hepatitis B virus envelope s antibody (anti-HBs), hepatitis B virus envelope e antigen (HBe-Ag), anti-hepatitis B virus envelope e antigen antibody (anti-HBe), anti-hepatitis B virus core antibody (anti-HBc), severe acute respiratory syndrome virus (SARS), PAI-1, phenytoin, phenobarbital, carbamazepine, valproic acid, theophylline, thymus and activation-regulated chemokine (TARC), soluble interleukin-2 receptor (sIL-2R), and pulmonary surfactant protein D (SP-D).

Additional examples of the measurement target substance that is measured in the present invention include anti-Treponema (Treponema Pallidum) antibodies, anti-cyclic citrullinated peptide (CCP) antibodies, anti-Helicobacter pylori antibodies, and antibodies such as IgG and IgA against viruses such as hepatitis, measles, and leukemia. In the technique described in Patent Document 3, there is a risk that these antibodies may also be degraded by the reducing agent. In contrast, the IgM-specific degrading enzyme used in the present invention does not degrade these antibodies. Accordingly, the IgM-specific degrading enzyme is particularly useful for the technique described in Patent Document 3 in those cases where the measurement target substance is an antibody.

### [Non-Specific Reaction Inhibitor]

In the present invention, the expression "inhibit non-specific reactions" means to act on factors that cause the above-mentioned non-specific reactions in biological samples (also known as non-specific factors, non-specific causative substances, or non-specific reaction substances), thereby inhibiting any effects of reactions other than antigen-antibody reactions on the measurements. Accordingly, in the present invention, a determination as to whether or not a candidate substance as a non-specific reaction inhibitor has an inhibitory effect on non-specific reactions can be made by comparing the measured values with and without the addition of the candidate substance against a measured value (hereinafter referred to as the control method measured value) obtained, for example, by conducting the measurement using a measurement method that employs a B/F separation (a method that includes a washing step, and is less susceptible to the effects of non-specific reaction substances (in the examples, the LBA method or CLEIA method)) as a reference, and ascertaining whether the measured value containing the candidate substance approaches the control method measured value. In other words, using the target measurement method, if the measured value obtained in the case where the candidate substance is added is closer to the control method measured value than the measured value obtained when the candidate substance is not added, a determination can be made that the candidate substance has a non-specific reaction inhibitory effect in the measurement method, meaning the candidate substance can be used as a non-specific reaction inhibitor.

The non-specific reaction inhibitor of the present invention targets both factors that cause a so-called positive measurement error in which the measurement target substance is determined to have a higher content than the actual content as a result of some component contained in the biological sample, and factors that cause a so-called negative measurement error in which the measurement target substance is determined to have a lower content than the actual content. Among these, the inhibitor is particularly effective against non-specific factors that cannot be inhibited by commercially available non-specific reaction inhibitors such as HBR-1. Further, the inhibitor is also effective against non-specific factors that cause positive measurement errors in which the measured value becomes abnormally high, and negative measurement errors in which the measured value becomes abnormally low, so-called deviant specimens. In the present invention, various causative substances that cause non-specific reactions may be exemplified, but it is preferable that the IgM-specific degrading enzyme degrades at least a portion or all of the immunoglobulin M (IgM), or a portion or all of the IgM and causative substances that have similar structures. As a result of this degradation reaction, the non-specific reaction inhibitor of the present invention can inhibit non-specific reactions derived from the causative substance.

The non-specific reaction inhibitor of the present invention may contain a substance which, based on the determination described above, is capable of inhibiting a reaction caused by a non-specific factor derived from the sample, and contains at least an IgM-specific degrading enzyme as an active ingredient. The non-specific reaction inhibitor of the present invention may employ a configuration in which the IgM-specific degrading enzyme is included in the above-mentioned reagent for immunological measurement as the non-specific reaction inhibitor.

The non-specific reaction inhibitor of the present invention may also contain buffers, proteins, peptides, amino acids, nucleic acids, lipids, phospholipids, sugars, glycoproteins, glycolipids, inorganic salts, polymer compounds, surfactants, other non-specific reaction inhibitors, and preservatives and the like, provided they do not interfere with the non-specific reaction inhibitory effect of the IgM-specific degrading enzyme. The other non-specific reaction inhibitors may be any inhibitors that have a non-specific reaction inhibitory effect, and include, but are not limited to, anti-IgM antibodies, polymer compounds, and modified antibodies in which a portion or all of the variable region of the L chain or H chain of a specific antibody has been modified. Moreover, when an anti-IgM antibody and an IgM-specific degrading enzyme are used in combination, the anti-IgM antibody and the enzyme may exist in a state where they are bound together, either directly or indirectly.

### [Method for Inhibiting Non-Specific Reactions]

A method for inhibiting non-specific reactions according to the present invention is a method for inhibiting non-specific reactions caused by the sample by conducting an antigen-antibody reaction at least once or more in the presence of the IgM-specific degrading enzyme. Further, the method for inhibiting non-specific reactions may also be termed a method for reducing measurement error.

The present invention is described below in further detail using a series of examples, but the present invention is not limited to the following examples. Examples

### <Non-Specific Reaction Inhibition in LTIA Method: Measurement of PCT>

The concentration of procalcitonin (PCT) contained in a sample (specimen) was measured in the following manner using the LTIA method.

Human serum specimens 1 and 2 from a plurality of individuals (two individuals) were used as samples. Specimen 1 (the control specimen) was a sample having a measured value using the LTIA method close to the measured value using the LBA method (Liquid-phase Binding Assay) (Reference Example 1). Specimen 2 (deviant specimen) was a sample exhibiting a non-specific reaction, and having a measured value using the LTIA method that deviates significantly from the measured value from Reference Example 1 using the LBA method. The IgM concentrations in the specimens was 62 mg/dL in specimen 1 and 88 mg/dL in specimen 2.

### [Reference Example 1] Measurement using the LBA Method

### 1. Measurement Method

### 1-1. Measurement Reagent

µTASWako (a registered trademark) PCT-i50 (manufactured by FUJIFILM Wako Pure Chemical Corporation)

### 1-2. Samples

Specimens 1 and 2

### 1-3 Measurement Procedure

Measurements were conducted using µTASWako (a registered trademark) i50 (manufactured by FUJIFILM Wako Pure Chemical Corporation) in accordance with the documentation included with the measurement reagent.

### 2. Measurement Results

The measurement results are shown in Table 1.

In the LBA method used in Reference Example 1, a B/F separation was conducted by isotachophoresis. As a result, the LBA method is a measurement method that is less susceptible to the effects of non-specific reactions derived from the sample.

### [Comparative Example 1] Measurement using the LTIA Method: PBS Buffer Solution Addition to Sample (No Additives)

### 1. Measurement Method

### 1-1. Measurement Reagent

A first regent and a second reagent were prepared in accordance with the methods described below.

### First Reagent

A first reagent base liquid was prepared with the following composition.
- 100 mM Bis-Tris-HCl (pH 6.5)
- 600 mM NaCl
- 0.2% BSA

### Second Reagent

- 5mM MOPS-NaOH (pH 7.0)
- Anti-human PCT monoclonal antibody-sensitized latex (two types)

The anti-human PCT monoclonal antibodies used were commercially available PCT antigens, and were acquired using methods well known to those skilled in the art. The anti-human PCT monoclonal antibody-sensitized latex was prepared with reference to the method disclosed in Japanese Unexamined Patent Application, First Publication No. 2017-181377. Specifically, to a 1.0% latex solution (5 mM Tris buffer solution (hereinafter referred to as Tris-HCl or simply Tris) (pH 8.5)) with an average particle size of 0.3 µm was added and stirred an equivalent volume of an anti-human PCT monoclonal antibody solution diluted to 0.36 mg/mL with 5 mM Tris-HCl (pH 8.5). An equal volume of 5 mM Tris-HCl (pH 8.5) containing 0.5% BSA was then added and stirred to prepare an anti-human PCT monoclonal antibody-sensitized latex particle solution.
This latex particle solution was diluted with 5 mM MOPS-NaOH (pH 7.0) to achieve an absorbance of about 5.5 OD at 600 nm, and the resulting solution was used as the second reagent.

### 1-2. Specimen Pretreatment Liquid

A PBS buffer solution (pH 7.4) was used.

### 1-3. Samples

Samples prepared by adding the PBS buffer solution used as the specimen pretreatment liquid to the specimens (serum samples) 1 and 2 described in Reference Example 1 in a volumetric ratio of 5:1 and conducting reaction at 37°C for 30 minutes were measured.

### 1-4. Measurement Procedure

The sample of each specimen, the first reagent, and the second reagent were mixed, and the PCT concentration in the sample was measured using a Hitachi 3500 automatic analyzer. Specifically, 120 µL of the first reagent was added to 12 µL of the sample and incubated at 37°C for 5 minutes. Subsequently, 40 µL of the second reagent was added and stirred. The change in absorbance associated with agglutination formation was measured over the subsequent 5 minutes at a main wavelength of 570 nm and a secondary wavelength of 800 nm. The measured value was calculated by applying the amount of change in the absorbance to a calibration curve obtained by measuring standard substances of known concentration.

### [Example 1] Measurement using the LTIA Method: IgM-Specific Degrading Enzyme Addition to Sample

### 1. Measurement Method

With the exception of using a PBS buffer solution containing 40 U/µL of the IgM-specific degrading enzyme IgM BRAZOR (manufactured by Genovis Inc.) for the specimen pretreatment liquid, measurements were conducted using the same method as Comparative Example 1.

### 2. Measurement Results

The measurement results are shown in Table 1. The units for the numerical values for the measurement results are ng/mL.

**[Table 1]**

| | Specimen number | IgM concentration mg/dL | Reference Example 1 | Example 1 | Comparative Example 1 |
|---|---|---|---|---|---|
| | | | LBA method | LTIA method | LTIA method |
| | | | - | IgM BRAZOR addition | PBS buffer solution addition |
| Control specimen | 1 | 62 | 5.06 | 5.10 | 5.04 |
| Deviant specimen | 2 | 88 | 0.10 | 0.02 | 1.68 |

### (1) The measurement results for the control specimen (specimen 1) were investigated.

For the control specimen 1, the measurement results for Reference Example 1, Example 1 and Comparative Example 1 were substantially equal. Based on these results, it was evident that addition of the IgMBRAZOR had no effect on the measured value for the specimen exhibiting no non-specific reactions.

### (2) The measurement results for the deviant specimen (specimen 2) were investigated.

The measured value for the specimen 2 was 0.10 ng/mL for Reference Example 1. The measured value for Comparative Example 1 was 1.68 ng/mL, a deviation from the measured value of Reference Example 1. In contrast, the measured value for Example 1 was 0.02 ng/mL, closer to the value for Reference Example 1.

The above results indicated that by using an IgM-specific degrading enzyme in the immunological measurement method, non-specific reactions derived from the sample could be inhibited.

### [Comparative Example 2] Measurement using the LTIA Method: Semi-Alkaline Protease Addition to Sample

With the exception of using a specimen pretreatment liquid prepared by diluting a proteinase from Aspergillus melleus, 5G (manufactured by Sigma-Aldrich Co., Ltd.) with a PBS buffer solution to achieve a concentration of 0.4 mg/mL, measurements were conducted using the same method as Example 1. The measurement results are shown in Table 2.

### [Comparative Example 3] Measurement using the LTIA Method: Semi-Alkaline Protease Addition to Sample

With the exception of altering the concentration of the enzyme contained in the specimen pretreatment liquid to 2.0 mg/mL, measurements were conducted using the same method as Comparative Example 2. The measurement results are shown in Table 2.

### [Comparative Example 4] Measurement using the LTIA Method: Proteinase K Addition to Sample

With the exceptions of changing the enzyme used to Proteinase K, recombinant, PCR grade, Lyophilizate from Pichia pastoris (manufactured by Roche AG), and using a solution prepared by diluting that enzyme with a PBS buffer solution to achieve a concentration of 0.4 mg/mL, measurements were conducted using the same method as Comparative Example 2. The measurement results are shown in Table 2.

### [Comparative Example 5] Measurement using the LTIA Method: Proteinase K Addition to Sample

With the exception of altering the concentration of the enzyme contained in the specimen pretreatment liquid to 2.0 mg/mL, measurements were conducted using the same method as Comparative Example 4. The measurement results are shown in Table 2.

The units for the numerical values for the measurement results shown in Table 2 are ng/mL.

**[Table 2]**

| | Specimen number | IgM concentration mg/dL | Example 1 | Comparative Example 1 | Comparative Example 2 | Comparative Example 3 | Comparative Example 4 | Comparative Example 5 |
|---|---|---|---|---|---|---|---|---|
| | | | LTIA method | LTIA method | LTIA method | LTIA method | LTIA method | LTIA method |
| | | | IgM BRAZOR addition | PBS buffer solution addition | Semi-alkaline protease addition 0.4 mg/mL | Semi-alkaline protease addition 2.0 mg/mL | Proteinase K addition 0.4 mg/mL | Proteinase K addition 2.0 mg/mL |
| Control Specimen | 1 | 62 | 5.10 | 5.04 | 0.44 | 0.08 | 0.17 | 0.08 |
| Deviant Specimen | 2 | 88 | 0.02 | 1.68 | 1.27 | - | 1.23 | - |

In the case of the control specimen 1, it is evident that the measured values for Comparative Examples 2 to 5 were markedly lower than the value for Example 1. The decrease was particularly marked when the enzyme concentration was high. It is thought that when a commercially available protease different from the IgM-specific degrading enzyme was used, the protease degraded the PCT antigen in the control specimen, resulting in a significant reduction in the measured value. In the deviant sample 2, the measured values in Comparative Examples 2 and 4 were higher than that in Example 1. It is thought that even though a commercially available protease different from the IgM-specific degrading enzyme was used, that protease was unable to degrade the non-specific causative substances in the deviant specimen, resulting in a higher measured value. Based on the above results, it can be stated that in the immunological measurement method, by using an IgM-specific degrading enzyme, non-specific reactions derived from the sample were able to be inhibited, without degrading the PCT antigen representing the measurement target substance in the sample.

### <Non-Specific Reaction Inhibition in LTIA Method: Measurement of sIL-2R>

The concentration of soluble Interleukin 2 receptor (sIL-2R) contained in a sample (specimen) was measured by the LTIA method in the following manner.

Human serum specimens 4 to 6 from a plurality of individuals (three individuals) were used as samples. Specimen 4 (the control specimen) was a sample having a measured value using the LTIA method close to the measured value using the Chemiluminescent Enzyme Immunoassay method (CLEIA method) ((Reference Example 2). Specimens 5 and 6 (deviant specimens) were samples exhibiting a non-specific reaction, each having a measured value using the LTIA method that deviates significantly from the measured value from Reference Example 2 using the CLEIA method. The IgM concentrations in the specimens were 76 mg/dL in specimen 4, 71 mg/dL in specimen 5, and 532 mg/dL in specimen 6.

### [Reference Example 2] Measurement using the CLEIA Method

### 1. Measurement Method

### 1-1. Measurement Reagent

Lumipulse Presto (a registered trademark) IL-2R (manufactured by FUJIREBIO Inc.)

### 1-2. Samples

Specimens 4 to 6

### 1-3 Measurement Procedure

Measurements were conducted using Lumipulse (a registered trademark) -L2400 (manufactured by FUJIREBIO Inc.) in accordance with the documentation included with the measurement reagent.

### 2. Measurement Results

The measurement results are shown in Table 3.

In the CLEIA method used in Reference Example 2, a B/F separation was conducted, and a washing step was included. As a result, the CLEIA method is a measurement method that is less susceptible to the effects of non-specific reactions derived from the sample.

### [Comparative Example 6] Measurement using the LTIA Method: PBS Buffer Solution Addition to Sample (No Additives)

### 1. Measurement Method

### 1-1. Measurement Reagent

A first regent and a second reagent were prepared in accordance with the methods disclosed in Japanese Unexamined Patent Application, First Publication No. 2017-181377.

### 1-2. Specimen Pretreatment Liquid

A PBS buffer solution (pH 7.4) was used.

### 1-3. Samples

Samples prepared by adding the PBS buffer solution used as the specimen pretreatment liquid to the specimens (serum samples) 4 to 6 described in Reference Example 2 in a volumetric ratio of 5:1 and conducting reaction at 37°C for one hour were measured.

### 1-4. Measurement Procedure

The sample of each specimen, the first reagent, and the second reagent were mixed, and the sIL-2R concentration in the sample was measured using a Hitachi 7180 automatic analyzer. Specifically, 120 µL of the first reagent was added to 5.6 µL of the sample and incubated at 37°C for 5 minutes. Subsequently, 40 µL of the second reagent was added and stirred. The change in absorbance associated with agglutination formation was measured over the subsequent 5 minutes at a main wavelength of 570 nm and a secondary wavelength of 800 nm. The measured value was calculated by applying the amount of change in the absorbance to a calibration curve obtained by measuring standard substances of known concentration.

### 2. Measurement Results

The measurement results are shown in Table 3. The units for the numerical values for the measurement results are U/mL.

### [Example 2] Measurement using the LTIA Method: IgM-Specific Degrading Enzyme Addition to Sample

With the exception of using a PBS buffer solution containing 40 U/µL of IgM BRAZOR (manufactured by Genovis Inc.) for the specimen pretreatment liquid, measurements were conducted using the same method as Comparative Example 6. The measurement results are shown in Table 3.

**[Table 3]**

| | Specimen number | IgM concentration mg/dL | Reference Example 2 | Example 2 | Comparative Example 6 |
|---|---|---|---|---|---|
| | | | CLEIA method | LTIA method | LTIA method |
| | | | - | IgM BRAZOR addition | PBS buffer solution addition |
| Control specimen | 4 | 76 | 1520 | 1464 | 1462 |
| Deviant specimens | 5 | 71 | 306 | 408 | 556 |
| | 6 | 532 | 591 | 830 | 905 |

Based on the results for Reference Example 2, Example 2 and Comparative Example 6, the non-specific reaction inhibitory effect of the IgM-specific degrading enzyme was examined.

### (1) The measurement results for the control specimen (specimen 4) were investigated.

For the control specimen, the measurement results for Reference Example 2, Example 2 and Comparative Example 6 were substantially equal. Based on these results, it was evident that addition of the IgMBRAZOR had no effect on the measured value for the specimen exhibiting no non-specific reactions.

### (2) The measurement results for the deviant specimens (specimens 5 and 6) were investigated.

The measured value for the specimen 5 was 306 U/mL for Reference Example 2. The measured value for Comparative Example 6 was 556 U/mL, a deviation from the measured value of Reference Example 2. On the other hand, the measured value for Example 2 was 408 U/mL, closer to the value for Reference Example 2.

A similar trend was observed for Specimen 6. The measured value for the specimen 6 was 591 U/mL for Reference Example 2. The value for Comparative Example 6 was 905 U/mL, a significant deviation from the measured value of Reference Example 2. On the other hand, the measured value for Example 2 was 830 U/mL, closer to the value for Reference Example 2.

The above results indicated that by using an IgM-specific degrading enzyme in the immunological measurement method, non-specific reactions derived from the sample could be inhibited. The deviant samples of these tests represent specimens for which a satisfactory inhibitory effect has been unattainable using existing non-specific reaction inhibitors such as anti-IgM antibodies and the like, and the non-specific reaction inhibition method of the present invention using an IgM-specific degrading enzyme represents the first time these non-specific reactions have been able to be inhibited.

## Claims

1. An immunological measurement method for measuring a measurement target substance in a sample, wherein
an antigen-antibody reaction is conducted at least once or more in presence of an enzyme that specifically degrades Immunoglobulin M.

2. The immunological measurement method according to Claim 1, wherein the enzyme is a protease.

3. The immunological measurement method according to Claim 2, wherein the protease is a proteinase or a peptidase.

4. The immunological measurement method according to Claim 1 or 2, wherein the enzyme is an enzyme that cleaves a portion of an IgM constant region including CHµ1 to CHµ4.

5. The immunological measurement method according to Claim 1 or 2, wherein the enzyme is an enzyme that acts on IgM in the sample and degrades the IgM into a F(ab')₂ fragment and a Fc fragment.

6. The immunological measurement method according to Claim 1 or 2, wherein the immunological measurement method is a latex turbidimetric immunoassay method.

7. The immunological measurement method according to Claim 6, wherein in the immunological measurement method, the measurement target substance is an antigen or an antibody.

8. A reagent for immunological measurement used in an immunological measurement method for measuring a measurement target substance in a sample, wherein
the reagent contains an enzyme that specifically degrades IgM.

9. The reagent for immunological measurement according to Claim 8, wherein the enzyme is a protease.

10. The reagent for immunological measurement according to Claim 9, wherein the protease is a proteinase or a peptidase.

11. The reagent for immunological measurement according to Claim 8 or 9, wherein the enzyme is an enzyme that cleaves a portion of an IgM constant region including CHµ1 to CHµ4.

12. The reagent for immunological measurement according to Claim 8 or 9, wherein the enzyme is an enzyme that acts on IgM in the sample and degrades the IgM into a F(ab')₂ fragment and a Fc fragment.

13. The reagent for immunological measurement according to Claim 8 or 9, wherein the reagent for immunological measurement is a reagent for use in a latex turbidimetric immunoassay method.

14. The reagent for immunological measurement according to Claim 13, wherein the measurement target substance in the immunological measurement method is an antigen or an antibody.

15. A specimen pretreatment liquid for immunological measurement containing an enzyme that specifically degrades IgM.

16. The specimen pretreatment liquid for immunological measurement according to Claim 15, wherein the enzyme is a protease.

17. The specimen pretreatment liquid for immunological measurement according to Claim 16, wherein the protease is a proteinase or a peptidase.

18. The specimen pretreatment liquid for immunological measurement according to Claim 15 or 16, wherein the enzyme is an enzyme that cleaves a portion of an IgM constant region including CHµ1 to CHµ4.

19. The specimen pretreatment liquid for immunological measurement according to Claim 15 or 16, wherein the enzyme is an enzyme that acts on IgM in the sample and degrades the IgM into a F(ab')₂ fragment and a Fc fragment.

20. The specimen pretreatment liquid for immunological measurement according to Claim 15 or 16, wherein the specimen pretreatment liquid for immunological measurement is a liquid for use in a latex turbidimetric immunoassay method.

21. A reagent kit for immunological measurement used in an immunological measurement method for measuring a measurement target substance in a sample, wherein the reagent kit contains an enzyme that specifically degrades IgM.

22. The reagent kit for immunological measurement according to Claim 21, wherein the enzyme is a protease.

23. The reagent kit for immunological measurement according to Claim 22, wherein the protease is a proteinase or a peptidase.

24. The reagent kit for immunological measurement according to Claim 21 or 22, wherein the enzyme is an enzyme that cleaves a portion of an IgM constant region including CHµ1 to CHµ4.

25. The reagent kit for immunological measurement according to Claim 21 or 22, wherein the enzyme is an enzyme that acts on the IgM in the sample and degrades the IgM into a F(ab')₂ fragment and a Fc fragment.

26. A non-specific reaction inhibitor containing an enzyme that specifically degrades IgM.
